# EUROPEAN PATENT APPLICATION

(11) **EP 1 338 607 A2**
(43) Date of publication of application: **27.08.2003**
(21) Application number: 03004818.5
(22) Date of filing: 16.07.1991
(51) Int. Cl.: C07K 14/22, C12N 15/31, C12N 15/11, A61K 38/16, A61K 39/095, C12Q 1/00, C07K 16/12

(54) **Antigenic iron repressible proteins from n. meningitidis related to the heolysin family of toxins**

(30) Priority: 16.07.1990 US 552649
(62) Divisional of application: 91913698.6
(71) Applicant: UNIVERSITY OF NORTH CAROLINA AT CHAPEL HILL, Chapel Hill, North Carolina 27514 (US)
(72) Inventor: Sparling, Frederick P., Moncure, NC 27559 (US); Thompson, Stuart Alan, Carrboro, NC 27510 (US)
(74) Representative: Grünecker, Kinkeldey, Stockmair & Schwanhäusser Anwaltssozietät

(57) **Abstract**

The present invention is directed to antigenic polypeptides isolated from Neisseria meningitidis, antibodies raised against the polypeptides, vaccines containing the polypeptides and DNA encoding the polypeptides.

## Description

The present invention is directed to antigenic polypeptides isolated from Neisseria meninaitidis, antibodies raised against the polypeptides, vaccines containing the polypeptides and DNA encoding the polypeptides. The polypeptides are members of the hemolysin family of toxins, a typical member of which is alpha-hemolysin from E. coli.

Bacterial pathogenesis is a complicated and often poorly understood process. Many pathogenic bacteria secrete toxins that impair the metabolism and function of animal cells. Various classes of molecules constitute such toxins.

An example of a protein toxin is found in pathogenic E. coli strains that cause extra-intestinal infections in humans. Such infections are characterized by the lysis of mammalian erythrocytes. The hemolytic activity is due to a class of toxins known as hemolysin. The class includes alpha-hemolysin and beta-hemolysin; see Welch et al, Infection and Immunity 42, 178-186 (1983).

Another protein toxin is adenylate cyclase, which is found in Bordetella pertussis and Bacillus anthracis, and which impairs functions of professional phagocytes. These bacteria are responsible for whooping cough and anthrax, respectively.

A third class of protein toxins from pathogenic bacteria are the leukotoxins, which are found in Actinobacillus actinomycetemcomitans, which is the etiologic agent of localized juvenile periodontitis, and Pasteurella haemolytica, which kills bovine leukocytes.

Interestingly, the adenylate cyclase from B. pertussis and B. anthracis and the leukotoxins from A. actinomycetemcomitans and P. haemolytica have amino acid sequences that exhibit considerable homology with that of alpha-hemolysin from E. Coli; see Glaser et al, Molecular Biology 2, 19-30 (1988) and Kolodrubetz et al, Infection and Immunity 57, 1465-1469 (1989). Apparently, there is a class of toxins found in various genera of bacteria. The amino acid sequence of this family of cytotoxins is characterized by a highly repeated nine amino acid motif, LxGGxGNDx, wherein x represents any amino acid. For the purposes of this specification, this family of toxins will be referred to as the hemolysin family of toxins.

It should be understood that "hemolysin family of toxins" is a generic name familiar to those in the art, and is not meant to imply that all members are hemolytic, or, for that matter, cytotoxic, although most are. Membership in the family depends on the existence of homology in the amino acid sequence, as defined below. In addition to those mentioned above, homologous proteins have also been found in Serratia and Proteus, although it is not certain whether these members of the hemolysin family are, in fact, cytotoxic.

Little is known about the intriguing and sometimes fatal bacteria Neisseria meninaitidis, which is responsible for spinal meningitis and septic shock. N. meningitidis and the diseases it causes have been reviewed by Paterson in "Neisseria meningitidis and Meningococcal Disease" in Biologic and Clinical Basis of Infectious Diseases, W. B. Saunders Company, Chapter 43 (1980).

The genotype of N. meningitidis is very similar to that of N. gonorrhoeae, although the phenotype is quite different. It is often important to distinguish between these Neisseria species. Immunologic speciation is often difficult due to a lack of sufficient amounts of group-specific antigens.

N. meningitidis exists as various serotypes, the prevalence of which varies with time and location. The serotypes include A, B, C, D, X, Y, Z; 29-E and W-135.

The three most important known antigenic and/or toxic constituents of N. meninaitidis infections are a capsular polysaccharide, a lipopolysaccharide-endotoxin cell wall complex and a Neisseria-specific protein. The capsular polysaccharide is a major _virulence factor that enables meningococci to resist phagocytosis by segmented neutrophils.

Vaccines containing meningococcal polysaccharides are used against some of the serotypes of N. meningitidis. For example, protection against the A, C, Y and W-135 serotypes is afforded by polysaccharide vaccines. Such vaccines are, however, inadequate for general protection against infection against N. meninaitidis. For example, the immune response of serotypes A and C to polysaccharide vaccines is poor, especially in children under two years old, who constitute the group most susceptible to meningococcal disease. Moreover, no effective vaccine exists for serotype B, possibly because the group B capsular polysaccharide is relatively non-immunogenic.

It is apparent that much needs to be learned about the pathology of N. meningitidis. Possibly, additional understanding of this pathogen will lead to the discovery of useful vaccines in general for more serotypes than are currently available.

For example, it is not known why the colonization of the respiratory tract by N. meninaitidis progresses to acute meningococcal disease and sometimes death in an occasional individual, whereas it does not do so in the great majority of others who are apparently at comparable risk. The amount of neither capsular polysaccharide nor lipopolysaccharide-endotoxin complex correlates with the seriousness of this disease. Exposure to microorganisms with antigenic constituents that cross-react with capsular polysaccharides of N. meningitidis has been proposed as an explanation; see Paterson, id.

Other explanations are also possible. For example, cross-immunity to antigens other than capsular polysaccharides cannot be ruled out. It is interesting to note in this regard that there are no known protein toxins associated with N. meningitidis. One reason for this may be that N. meningitidis is often cultured in vitro under iron-rich conditions that do not exist in a human host. It is known, however, that some meningococcal proteins are iron-repressed and are not observed in vitro, although they are expressed in vivo. See Black et al., Infection and Immunity 54, 710-713 (1986) and Brener et al, ibid. 33, 59-66 (1981).

One problem addressed by the present invention is the discovery of antigenic polypeptides and DNA sequences that are capable of identifying N. meninaitidis and distinguishing it from N. gonorrhoeae. Another problem addressed by the present invention is the discovery of proteins capable of producing antibodies effective against meningococcal disease.

### SUMMARY OF THE INVENTION

These and other problems as will be apparent to those having ordinary skill in the art have been solved by providing an isolated, antigenic polypeptide comprising a segment having at least fifty amino acid residues, wherein the amino acid sequence of the segment is present in N. meningitidis, and wherein the amino acid sequence is different from, but substantially homologous with, the amino acid sequence of a segment of a member of the hemolysin family of toxins. Another way of defining the polypeptide is to say that it is an isolated polypeptide comprising a segment having an amino acid sequence present in N. meningitidis wherein the amino acid sequence consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x, independently, represents any single amino acid residue.

The invention further includes antigenic fragments of such polypeptides, antibodies raised against such polypeptides, nucleotide sequences encoding such polypeptides, and vaccines containing such polypeptides.

### DETAILED DESCRIPTION OF THE INVENTION

### The Polypeptide Segment

It has unexpectedly been discovered that when N. meninaitidis is grown under iron-limiting conditions, a polypeptide comprising a segment having an amino acid sequence that is different from, but substantially homologous with, a segment of the hemolysin family of toxins is expressed. Monoclonal antibodies raised against the polypeptide found in N. meninaitidis, such as A4.85 (see below), cross-react in Western blots with alpha-hemolysin (HlyA) produced by the hlyA gene in E. coli and adenylate cyclase produced in Bordetella pertusssis.

The hemolysin family of toxins, as used herein, includes the homologous, cytotoxic or proteolytic polypeptides found in bacteria of the genera Escherichia, Serratia, Pasteurella, Proteus, Actinobacillus, and Bordetella. The family specifically includes alpha-hemolysin, leukotoxin, and adenylate cyclase.

Determinations whether two amino acid sequences are substantially homologous are, for the purpose of the present specification, based on FASTA searches in accordance with Pearson and Lipman, Proc. Natl. Acad. Sci. USA 85, 2444-2448 (1988). A substantially homologous sequence in accordance with the present invention has at least 15% identity, preferably at least 20% identity and, more preferably, at least 25% identity in amino acid sequence when determined in accordance with the method of Pearson and Lipman, which is incorporated herein by reference.

The polypeptide of the present invention need not contain a segment that is identical to other members of the hemolysin family of toxins. The identity in accordance with the FASTA method may be as high as 90% or 95%, but when isolated from N. meningitidis normally does not exhibit identities greater than 40% or 50%.

The size of the polypeptide is not critical, as long as it contains a segment that is substantially homologous to a segment of a member of the hemolysin family of toxins. The substantially homologous segment has at least fifty amino acid residues, preferably at least 100 amino acid residues, and more preferably at least 200 amino acid residues. In this specification, the word "polypeptide" will be considered indistinguishable from words like "protein" and "peptide."

The segment of the meningococcal polypeptide that is substantially homologous to a segment of the hemolysin family of toxins contains the same nine amino acid motif that is characteristic of all the members of the hemolysin family of toxins. The consensus sequence is LxGGxGNDx, hereinafter hemolysin consensus sequence. The amino acid represented by x may be any single amino acid.

The polypeptide of the invention may be defined in terms of the hemolysin consensus sequence as well as by the substantial homology standard described above. For this purpose, a nine amino acid sequence is considered to be a hemolysin consensus sequence if it contains at least four, preferably at least five, and more preferably all six of the specifically defined amino acid residues (i.e. L-GG-GND-) at the correct position.

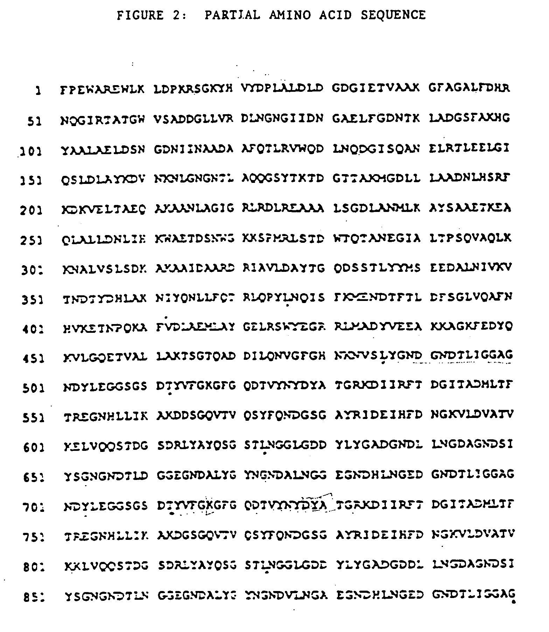

Referring to Figure 2, which, as described below, is a partial polypeptide isolated from N. meninaitidis, the homologous segment comprises three stretches of multiple repeats of the hemolysin consensus sequence, i.e. between amino acids 486 and 512, between amino acids 623 and 712, and from 823 to the end. There are 21 complete consensus sequences in Figure 2.

The polypeptides of the present invention contain segments that are present in N. meningitidis and that comprise at least three, preferably at least five, and more preferably at least ten hemolysin consensus sequences. The polypeptides of the invention may have as many as at least 21 hemolysin consensus sequences.

The polypeptide is isolated, which means that it is essentially free of other proteins, especially of other proteins from N. meninaitidis. Essentially free from other proteins means that it is at least 90%, preferably at least 95% and, more preferably, at least 98% free of other proteins.

Preferably, the polypeptide is essentially pure, which means that the polypeptide is free not only of other polypeptides, but also of other materials used in the isolation and identification of the polypeptide, such as, for example, sodium dodecyl sulfate and other detergents as well as nitrocellulose paper. The polypeptide is at least 90% free, preferably at least 95% free and, more preferably, at least 98% free of such materials.

The polypeptide of the present invention is antigenic, which means that the polypeptide induces specific antibodies in a mammal. Preferably, the polypeptide is immunogenic.

The polypeptide may be the entire polypeptide as it exists in N. meningitidis, or an antigenic, preferably immunogenic, fragment of the whole polypeptide. Antigenic and/or immunogenic fragments of antigenic and/or immunogenic polypeptides may be identified by methods known in the art. Usually, the antigenic fragment will comprise at least a portion of the segment having an amino acid sequence that is different from, but homologous, to the amino acid sequence of a segment of a polypeptide that is a member of the hemolysin family of toxins, or will comprise at least a portion of the segment having at least three, preferably at least five, and more preferably at least ten hemolysin consensus sequences.

### Preparation of the Polypeptide

The polypeptides of the present invention may be prepared by methods known in the art. Such methods include isolating the polypeptide directly from N. meningitidis; isolating or synthesizing DNA encoding the polypeptide and using the DNA to produce recombinant polypeptide; and synthesizing the polypeptide from individual amino acids.

The polypeptide or DNA encoding the polypeptide may be isolated from any serotype of N. meninaitidis. Such serotypes include A, B, C, D, X, Y, Z, 29-E and W-135.

Suitable sources of meningococcal strains from which the polypeptide and DNA encoding the polypeptide may be isolated are available. Such sources include the American Type Culture Collection (Bethesda, MD) and the Neisseria Repository (NAMRU, University of California, Berkeley). Suitable strains include FAM18 and FAM20 (Dyer et al, Microbial Pathogenesis 3, 351-363 (1987)), and FAM 19. Additional meningococcal strains are described by Schryvers and Morris in Infection and Immunity 56, 1144-1149 (1988).

The polypeptide may be isolated directly from N. meningitidis by methods known in the art. First, meningococcal outer membranes are isolated and prepared by known methods. The methods described by West and Sparling in Infect. Immun. 47, 388-394 (1985) and by Schryvers and Morris in Infect. Immun. 56, 1144-1149 (1988) are suitable.

The isolated membrane proteins may be solubilized by known methods, such as the addition of detergents. Commonly used detergents include Octyl-B-Glucoside, Chaps, Zwittergent 3.14 or Triton-X. The use of detergents to enhance solubility of membrane proteins is described by Jones et al. in Finby, Solubilization and Reconstitution of Membrane Proteins: A Practical Approach, IRL Press (1986), Helenius et al. in Biochim. Biophys. Acta 415, 29 (1975) and Hjelmeland and Chrambach, Methods Enzymol. 104, 305 (1984).

Proteins are isolated from the solubilized membrane fraction by standard methods. Some suitable methods include precipitation and liquid chromatographic protocols such as ion exchange, hydrophobic interaction and gel filtration. See, for example, Methods Enzymol. 182 (Guide to Protein Chemistry, Deutscher, Ed. Section VII) 309 (1990) and Scopes, Protein Purification. Springer-Verlag, New York (1987).

Alternatively, purified material is obtained by separating the protein on preparative SDS-PAGE gels, slicing out the band of interest and electroeluting the protein from the polyacrylamide matrix by methods known in the art. The detergent SDS is removed from the protein by known methods, such as by dialysis or the use of a suitable column, such as the Extracti-Gel column from Pierce.

The polypeptide may also be produced by isolating DNA that encodes the polypeptide; cloning the DNA in a suitable host; expressing the DNA in the host; and harvesting the polypeptide.

The first DNA encoding the polypeptide of the present invention was isolated by an immunoscreening method. Such methods are described by Maniatis et al in "Molecular Cloning: A Laboratory Manual," Cold Spring Harbor Laboratory Press, Cold Spring Harbor, New York (1982).

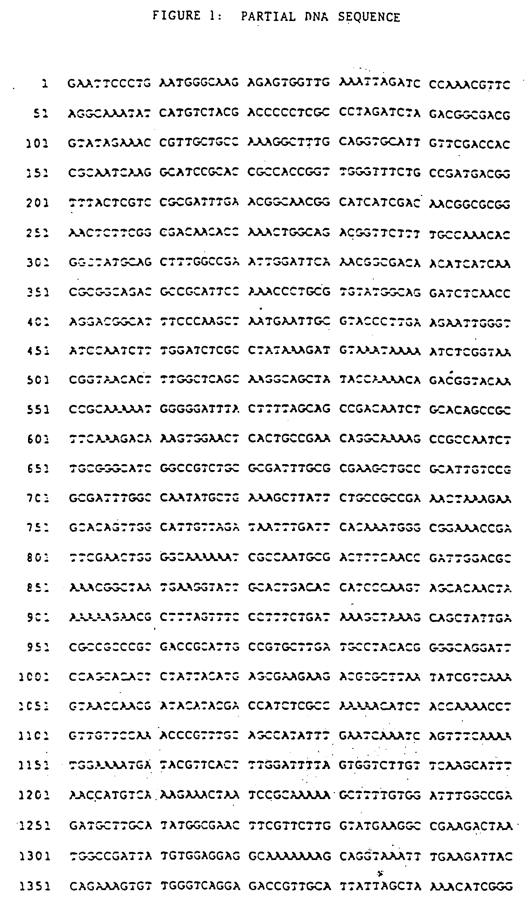

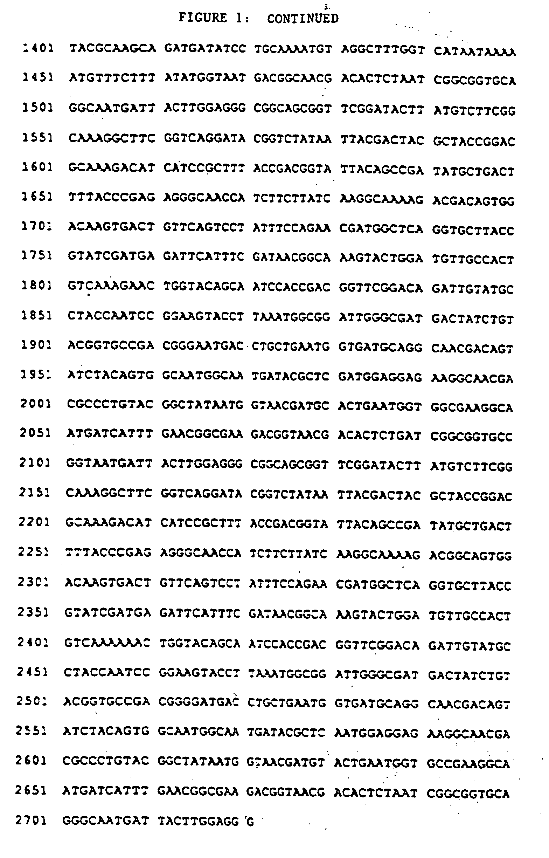

Briefly, monoclonal antibodies were generated against iron-stressed outer membrane proteins of N. meningitidis strain FAM20. One monoclonal antibody, A4.85, recognized several iron-regulated proteins in Western blots of the FAM20 outer membranes. A4.85 was used to isolate a clone from an FAM20 genomic library constructed in the expression vector lambda-gt11. The sequence of this clone was determined and used to clone adjacent genomic restriction fragments. The adjoined DNA sequence of this region contained a long open reading frame, which is shown as Figure 1. The amino acid sequence predicted from the nucleotide sequence of Figure 1 is shown as Figure 2.

A FASTA homology search in accordance with Pearson and Lipman, Proc. Natl. Acad. Sci. USA 85, 2444-2448 (1988) of the amino acid sequence deduced from the open reading frame (Figure 2) was performed. Surprisingly, the amino acid sequence exhibited substantial homology to several members of the hemolysin family of toxins, as discussed above.

As further evidence that the polypeptide isolated from N. meningitidis is a member of the hemolysin family of toxins, the antibody raised against, and used to isolate, the polypeptide shown as Figure 2, A4.85, cross-reacted strongly with alpha-hemolysin (HlyA) from E. coli and with adenylate cyclase produced in Bordetella pertusssis.

The immunoscreening method may be repeated in order to obtain additional fragments of the gene encoding the polypeptide of the invention or to obtain the gene encoding the entire polypeptide. It is, of course, not necessary to repeat the immunoscreening process. The entire gene or additional fragments of the gene are preferably isolated by using the known DNA sequence or fragments thereof as a probe. To do so, meningococcal DNA restriction fragments, either flanking the ends of the region already cloned or containing the entire region, are identified by Southern hybridization using labelled oligonucleotide probes derived from a previously determined sequence, such as that shown as Figure 1, or a fragment thereof.

The DNA obtained may be amplified by methods known in the art. One suitable method is the polymerase chain reaction (PCR) method described by Mullis et al in U.S. Patent 4,683,195 and by Sambrook, Fritsch and Maniatis (eds) in Molecular Cloning, A Laboratory Manual, Second Edition, Cold Spring Harbor Laboratory Press (1989). It is convenient to amplify the clones in the lambda-gt11 vectors using lambda-gt11-specific oligomers as the amplimers (available from Stratagene).

The restriction fragments are cloned into a suitable vector, such as a plasmid or bacteriophage, and sequenced in accordance with methods known in the art. A suitable sequencing method is the dideoxy chain terminating method described by Sanger et al in Proc. Natl. Acad. Sci. USA 74, 5463-5467 (1977). Suitable vectors and polymerases for sequencing are known. A suitable vector is the Bluescript vector of Stratagene. A suitable polymerase is Sequenase (United States Biochemical Corp., Cleveland, OH).

The DNA encoding the polypeptide of the invention may be used to express recombinant polypeptide in a wide variety of host cells using a wide variety of vectors. The host may be prokaryotic or eukaryotic. The DNA may be obtained from natural sources and, optionally, modified. The genes may also be synthesized in whole or in part.

Cloning vectors may comprise segments of chromosomal, non-chromosomal and synthetic DNA sequences. Some suitable prokaryotic vectors include plasmids from E. coli, such as colE1, pCR1, pBR322, pMB9, and RP4. Prokaryotic vectors also include derivatives of phage DNA such as M13, fd, and other filamentous single-stranded DNA phages.

Vectors for expressing proteins in bacteria, especially E.coli, are also known. Such vectors include pK233 (or any of the tac family of plasmids), T7, and lambda P_{L}. Examples of vectors that express fusion proteins include the PATH vectors described by Dieckmann and Tzagoloff in J. Biol. Chem. 260, 1513-1520 (1985). These vectors contain DNA sequences that encode anthranilate synthetase (TrpE) followed by a polylinker at the carboxy terminus. Other expression vector systems are based on beta-galactosidase (pEX) ; maltose binding protein (pMAL); and glutathione S-transferase (pGST) - see Gene 67, 31 (1988) and Peptide Research 3, 167 (1990).

Vectors useful in yeast are available. A suitable example is the 2u plasmid.

Suitable vectors for use in mammalian cells are also known. Such vectors include well-known derivatives of SV-40, adenovirus, retrovinis-derived DNA sequences and vectors derived from combination of plasmids and phage DNA.

Further eukaryotic expression vectors are known in the art (e.g., P.J. Southern and P. Berg, J. Mol. Appl. Genet. 1, 327-341 (1982); S. Subramani et al, Mol. Cell. Biol. 1, 854-864 (1981); R.J. Kaufmann and P.A. Sharp, "Amplification And Expression Of Sequences Cotransfected with A Modular Dihydrofolate Reductase Complementary DNA Gene," J. Mol. Biol. 159, 601-621 (1982); R.J. Kaufmann and P.A. Sharp, Mol. Cell. Biol. 159, 601-664 (1982); S.I. Scahill et al, "Expression And Characterization Of The Product Of A Human Immune Interferon DNA Gene In Chinese Hamster Ovary Cells," Proc. Natl. Acad. Sci. USA 80, 4654-4659 (1983); G. Urlaub and L.A. Chasin, Proc. Natl. Acad. Sci. USA 77, 4216-4220, (1980).

Useful expression hosts include well-known prokaryotic and eukaryotic cells. Some suitable prokaryotic hosts include, for example, E. coli, such as E. coli SG-936, E. coli HB 101, E. coli W3110, E. coli X1776, E. coli X2282, E. coli DHI, and E. coli MRCl, Pseudomonas, Bacillus, such as Bacillus subtilis, and Streptomyces. Suitable eukaryotic cells include yeasts and other fungi, insect, animal cells, such as COS cells and CHO cells, human cells and plant cells in tissue culture.

The expression vectors useful in the present invention contain at least one expression control sequence that is operatively linked to the DNA sequence or fragment to be expressed. The control sequence is inserted in the vector in order to control and to regulate the expression of the cloned DNA sequence. Examples of useful expression control sequences are the lac system, the trp system, the tac system, the trc system, major operator and promoter regions of phage lambda, the control region of fd coat protein, the glycolytic promoters of yeast, e.g., the promoter for 3-phosphoglycerate kinase, the promoters of yeast acid phosphatase, e.g., Pho5, the promoters of the yeast alpha-mating factors, and promoters derived from polyoma, adenovirus, retrovirus, and simian virus, e.g., the early and late promoters or SV40, and other sequences known to control the expression of genes of prokaryotic or eukaryotic cells and their viruses or combinations thereof.

The recombinant polypeptide is purified by methods known in the art. Suitable methods are described F. A. O. Marston, "The Purification of Eukaryotic Polypeptides Expressed in Escherichia coli," in DNA Cloning, D. M. Glover, Ed., Vol. III, IRL Press Limited, England (1987).

The polypeptide of the invention and DNA encoding the polypeptide may also be chemically synthesized from individual amino acid residues and nucleotides, respectively, by methods known in the art. Suitable methods for synthesizing the polypeptide are described by Stuart and Young in "Solid Phase Peptide Synthesis," Second Edition, Pierce Chemical Company (1984). Suitable methods for synthesizing DNA are described by Caruthers in Science 230, 281-285 (1985).

### VACCINES

A polypeptide comprising a segment having an amino acid sequence that is different from, but substantially homologous with, the amino acid sequence of a member of the hemolysin family of toxins is, unexpectedly, an antigen useful for protecting a mammal from infectious diseases caused by N. meningitidis. The mammal is typically a human.

To be useful, the antigen is non-toxic to the mammal being immunized. If the antigen is toxic, it may be detoxified by methods known in the art. Such methods include, for example, providing antigenic, non-toxic fragments of the entire polypeptide or detoxifying a polypeptide by, for example, binding the toxin to a carrier molecule that destroys toxicity,. but does not affect antigenicity. The carrier molecule is typically another polypeptide.

Preferably, an amino acid sequence of the antigen is present in a polypeptide found in N. meningitidis. The polypeptide or non-toxic, antigenic fragments useful in immunizing mammals may be made by methods known in the art, such as by isolation from N. meningitidis, production by recombinant DNA techniques, or chemical synthesis, as described above.

The length of the fragment is not critical as long as the fragment is antigenic and non-toxic. Therefore, the fragment should contain sufficient amino acid residues to define the epitope. Methods for isolating and identifying antigenic fragments from known antigenic polypeptides are described by Salfeld et al. in J. Virol. 63, 798-808 (1989) and by Isola et al. in J. Virol. 63, 2325-2334 (1989).

If the fragment defines the epitope, but is too short to be antigenic, it may be conjugated to a carrier molecule. Some suitable carrier molecules include keyhole limpet hemocyanin and bovine serum albumen. Conjugation may be carried out by methods known in the art. One such method is to combine a cysteine residue of the fragment with a cysteine residue on the carrier molecule.

The present invention further includes vaccine compositions for immunizing mammals, including humans, against infection by N. meningitidis. The vaccine comprises an immunogenic antigen as described above in a suitable carrier.' Suitable carriers include any of the standard pharmaceutically acceptable carriers, such as water, phosphate buffered saline solution, and emulsions.

The vaccine may include adjuvants, such as muramyl peptides, and lymphokines, such as interferon, interleukin-1 and interleukin-6. The antigen may be adsorbed on suitable particles, such as aluminum oxide particles, or encapsulated in liposomes, as is known in the art.

The invention further includes methods of immunizing host mammals, including humans, by administering the vaccine compositions described above to mammals in need of protection from diseases caused by N. meningitidis. The vaccine comprises an immunogenic polypeptide in a form that is non-toxic to mammals. The polypeptide comprises an amino acid sequence that is homologous with the amino acid sequence of a member of the hemolysin family of toxins. The amino acid sequence is preferably present in N. meningitidis, and is usually found in the outer membranes of N. meningitidis. Since, however, antibodies cross-react with the polypeptide of the invention and members of the hemolysin family of toxins from other genera of bacteria, the antigen in the vaccine composition may comprise an amino acid sequence in such other genera, such as from E. coli or B. pertussis.

The vaccine may be administered to a mammal by methods known in the art. Such methods include, for example, intravenous, intraperitoneal, subcutaneous, or intramuscular administration.

### Antibodies

The present invention provides antibodies raised against a polypeptide of the invention. The polypeptide comprises an amino acid sequence that defines an epitope, and is substantially homologous with the amino acid sequence of a member of the hemolysin family of toxins. The antibodies are preferably raised against a polypeptide comprising an amino acid sequence that is present in N. meninaitidis, and that is different from polypeptides that are members of the hemolysin family of toxins from other genera of bacteria.

The antibodies are preferably monoclonal. Monoclonal antibodies may be produced by methods known in the art. These methods include the immunological method described by Kohler and Milstein in Nature 256, 495-497 (1975) and the recombinant DNA method described by Huse et al in Science 246, 1275-1281 (1989).

Mammals, including humans, suffering from diseases caused by infection with N. meningitidis may be treated by administering antibodies specific to a member of the hemolysin family of toxins. Antibodies raised against a member of the hemolysin family of toxins from any genera of bacteria are suitable, although antibodies raised against a polypeptide comprising an amino acid sequence present in N. meninaitidis is preferred.

For therapeutic purposes, it is necessary for the antigenic polypeptides of the invention to produce neutralizing antibodies. Neutralizing antibodies are antibodies that significantly inhibit the growth of or kill the bacterial cells and/or significantly neutralize the toxin function of the polypeptide in vitro or in vivo. Growth of the bacteria is significantly inhibited or the toxin function of the polypeptide is significantly neutralized in vivo if the inhibition or neutralization is sufficient to prevent or reduce the symptoms of the disease of a mammal infected with the disease.

Neutralizing antibodies may also be used to produce anti-idiotypic antibodies useful as vaccines for immunizing mammals, including humans, suffering from diseases caused by infection with N. meningitidis. Anti-idiotypic antibodies are prepared in accordance with methods known in the art.

### NUCLEIC ACID MOLECULES

The present invention also includes isolated nucleic acid molecules that encode any of the polypeptides of the invention described above. The nucleic acid molecule may be DNA or RNA.

The utility of the nucleic acid molecule lies in its ability to be used as a probe for detecting N. meningitidis, as explained below, or to produce a polypeptide of the invention, as explained above. The nucleic acid molecule may be prepared by methods known in the art. Suitable methods include isolating the DNA from N. meningitidis or synthesizing the DNA in accordance with known procedures as described above.

### PROBES

The present invention further provides a method of detecting the presence of N. meningitidis in a sample. The method involves use of a probe that recognizes a polypeptide that is a member of the hemolysin family of toxins, and, in particular, a member of the hemolysin family of toxins present in N. meningitidis, or a gene encoding such a polypeptide. The probe recognizes N. meningitidis if present in the sample.

The probe may be an antibody, preferably a monoclonal antibody. The antibodies may be prepared as described above.

Methods are known for detecting polypeptides with antibodies. For example, a polypeptide may be immobilized on a solid support. Immobilization of the polypeptide may occur through an immobilized first antibody specific for the polypeptide. The immobilized first antibody is incubated with a sample suspected of containing the polypeptide. If present, the polypeptide binds to the first antibody.

A second antibody, also specific for the polypeptide, binds to the immobilized polypeptide. The second antibody may be labelled by methods known in the art. Non-immobilized materials are washed away, and the presence of immobilized label indicates the presence of the polypeptide. This and other immunoassays are described by David, et al., in U.S. Patent 4,376,110 assigned to Hybritech, Inc., La Jolla, California.

The probe may also be a nucleic acid molecule that recognizes a RNA or DNA molecule that encodes a member of the hemolysin family of toxins present in N. meningitidis. Methods for determining whether a nucleic acid molecule probe recognizes a specific nucleic acid molecule in a sample are known in the art. Generally, a labelled probe that is complementary to a nucleic acid sequence suspected of being in a sample is prepared. The presence of probe hybridized to the target nucleic acid molecule indicates the presence of the nucleic acid molecule. Suitable methods are described by Schneider et al in U.S. Patent 4,882,269, which is assigned to Princeton University, and by Segev in PCT Application WO 90/01069. The Schneider et al patent and the Segev application are both licensed to ImClone Systems Inc., New York City.

The probes described above are labelled in accordance with methods known in the art. Methods for labelling antibodies have been described, for example, by Hunter and Greenwood in Nature 144, 945 (1962) and by David et al in Biochemistry 13, 1014-1021 (1974). Additional methods for labelling antibodies have been described in U.S. patents 3,940,475 and 3,645,090. Methods for labelling oligonucleotide probes have been described, for example, by Leary et al, Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al, Nucl. Acids Res. (1985) 13:2399; and Meinkoth and Wahl, Anal. Biochem. (1984) 138:267.

The label may be radioactive. Some examples of useful radioactive labels include ³²P, ¹²⁵I, ¹³¹I, and ³H. Use of radioactive labels have been described in U.K. 2,034,323, U.S. 4,358,535, and U.S. 4,302,204.

Some examples of non-radioactive labels include enzymes, chromophors, atoms and molecules detectable by electron microscopy, and metal ions detectable by their magnetic properties.

Some useful enzymatic labels include enzymes that cause a detectable change in a substrate. Some useful enzymes and their substrates include, for example, horseradish peroxidase (pyrogallol and o-phenylenediamine), beta-galactosidase (fluorescein beta-D-galactopyranoside), and alkaline phosphatase (5-bromo-4-chloro-3-indolyl phosphate/nitro blue tetrazolium). The use of enzymatic labels have been described in U.K. 2,019,404, EP 63,879, and by Rotman, Proc. Natl. Acad. Sci., 47, 1981-1991 (1961).

Useful chromophores include, for example, fluorescent, chemiluminescent, and bioluminescent molecules, as well as dyes. Some specific chromophores useful in the present invention include, for example, fluorescein, rhodamine, Texas red, phycoerythrin, umbelliferone, luminol.

The labels may be conjugated to the antibody or nucleotide probe by methods that are well known in the art. The labels may be directly attached through a functional group on the probe. The probe either contains or can be caused to contain such a functional group. Some examples of suitable functional groups include, for example, amino, carboxyl, sulfhydryl, maleimide, isocyanate, isothiocyanate.

The label may also be conjugated to the probe by means of a ligand attached to the probe by a method described above and a receptor for that ligand attached to the label. Any of the known ligand-receptor combinations is suitable. The biotin-avidin combination is preferred.

The polypeptide of the invention may be used to detect the presence of antibodies specific for N. meninaitidis in a sample. The method comprises preparing a polypeptide containing a segment having an amino acid sequence that is substantially homologous to a member of the hemolysin family of toxins. The polypeptide may be prepared as described above. Preferably, the polypeptide comprises a segment having an amino acid sequence that is present in N. meningitidis.

The sample may, for example, be from a patient suspected of being infected with N. meningitidis. Suitable assays are known in the art, such as the standard ELISA protocol described by R.H. Kenneth, "Enzyme-Linked Antibody Assay with Cells Attached to Polyvinyl Chloride Plates" in Kenneth et al, Monoclonal Antibodies, Plenum Press, N.Y., page 376 (1981).

Briefly, plates are coated with antigenic polypeptide at a concentration sufficient to bind detectable amounts of the antibody. After incubating the plates with the polypeptide, the plates are blocked with a suitable blocking agent, such as, for example, 10% normal goat serum. The sample, such as patient sera, is added and titered to determine the endpoint. Positive and negative controls are added simultaneously to quantitate the amount of relevant antibody present in the unknown samples. Following incubation, the samples are probed with goat anti-human Ig conjugated to a suitable enzyme. The presence of anti-polypeptide antibodies in the sample is indicated by the presence of the enzyme.

Antibodies raised against polypeptides of the present invention are capable of recognizing N. meningitidis and distinguishing meningococcal cells from gonococcal cells in a sample. The A4.85 monoclonal antibody, for example, recognizes a polypeptide expressed by iron-stressed meningococcal cells. A4.85 does not, however, recognize any proteins in iron-stressed N. gonorrhoeae.

The antibodies may be labelled by known methods as described above. Assays for distinguishing iron-stressed meningococcal cells from iron-stressed gonococcal cells follow known formats, such as standard blot and ELISA formats.

### EXAMPLES

### Example 1. Isolation of A4.85 MAb

Bacterial outer membranes are prepared from iron-stressed cultures of Neisseria meninaitidis strain FAM20 as follows. FAM20 is inoculated into chelexed defined medium (CDM, West et al, J. Bacteriology 169, 3414 (1987)). This medium allows growth only until iron stores within the bacteria have been depleted. During this time, a set of proteins that are regulated by the availability of iron become expressed. Bacteria are harvested and outer membranes are prepared as described by Dyer et al in Infection and Immunity 56, 977 (1988).

Three to five BALB/c female mice are immunized with iron-stressed FAM20 outer membranes by either the intramuscular (im) or intraperitoneal (ip) routes. With the im route, 100 ug of antigen (Ag) is emulsified in complete Freund's adjuvant and injected on two different sites on day zero, followed by booster doses two weeks apart with the Ag now emulsified in incomplete Freund's adjuvant. The ip route involves immunization with 100 ug of Ag on days zero, 7, 14 and 28. Serum antibody levels are checked by either ELISA or Western blotting three days following the final boost to determine serum antibody levels. Mice are given a final boost ip on each of three consecutive days before the fusion. On the day of the fusion, mice are sacrificed by cervical dislocation and the spleens are removed aseptically. Spleen cells are extracted by teasing the cells out of the sac using two bent 19 ga needles. Extracted cells are resuspended to give single cell suspensions.

Mouse myeloma cells, SP2.0-AG14 (ATCC CRL 1581), that have been grown in Dulbecco's Modified Eagle's Medium/Ham's F-12 (DMEM/F12) supplemented with 15% fetal calf serum (FCS), are used as the fusion partner. Cells are mixed in a 10:1 ratio of spleen:myeloma cells and pelleted together in a 50 ml centrifuge tube. The supernatant is aspirated off leaving a dry pellet to which 1 ml of 50% polyethylene glycol (PEG) (prewarmed to 37°C) is added. The cells are gently resuspended and allowed to sit at room temperature for 2 minutes, after which 1 ml of DMEM/F12 without added sera is added and the cells gently resuspended. The cells are then further diluted and resuspended with 2, 4, 8 and 16 ml of DMEM/F12 added at two-minute intervals. The cells are then pelleted and the supernatant aspirated. Two ml of DMEM/F12 supplemented with 15% FCS is carefully added to avoid resuspension of the pellet and then incubated for one hour at 37°C.

At the end of the 1 hour incubation, the suspension is diluted to a final concentration of 1x10⁶ cells/ml. This suspension is then poured into tissue culture flasks and incubated overnight. The next day an equal volume of culture media supplemented with 2X HAT (hypoxanthine, aminopterin, thymidine) components are plated out into 96-well plates with 200 ul/well with 1x10⁵ spleen cells/well. Plated cells are fed every 4-5 days after the fusion by aspiration of half the media from the wells and addition of fresh 1xHAT media. The wells are scored for growth after 10-14 days, and growing wells are tested for presence of secreted antibody by screening the culture supernatants by either an ELISA or Western blotting. Wells that prove positive on assay are expanded for growth into 24-well culture dishes in culture media with HT supplements (no aminopterin) and re-tested. Those proving positive on re-testing are expanded further into larger tissue culture vessels and then cloned twice by limiting dilution.

A cell line (A4.85) that arose from a single mouse spleen cell was isolated. A4.85 produces a monoclonal antibody (MAb) that reacts with several protein species (70 kilodaltons to several hundred kilodaltons in mass) on a Western blot of FAM20 outer membranes, each of whose synthesis is repressed by the presence of iron in the bacterial growth medium.

### Example 2A. Isolation of Genomic Clones

### A. Library construction

A library of Neisseria meningitidis strain FAM20 chromosomal DNA is constructed in the bacteriophage vector lambda-gtil as follows. FAM20 chromosomal DNA is isolated by standard methods (Maniatis et al, 1982). The DNA is sheared by sonication to fragment sizes of approximately 300-1000 bp. Synthetic EcoRI linkers are ligated to the ends of these molecules, followed by cleavage with EcoRI restriction endonuclease to generate EcoRI restriction sites at the end of each molecule. The resulting fragments are ligated with EcoRI-cleaved lambda-gt11 DNA (Maniatis et al, Molecular Cloning: A Laboratory Manual, Cold Spring Harbor Laboratory Press, Cold Spring Harbor, New York (1982)). The ligated DNA is packaged into lambda phage heads using lambda packaging extracts (Promega Corp., Madison, Wisconsin), according to manufacturer's instructions.

### B. Library Screening and Isolation of DNA

The library created above is screened with the A4.85 MAb to detect clones that express the epitope recognized by A4.85. 500,000 recombinant plaques from the lambda-gt11 expression library are screened by the method of Maniatis et al (1982). A pure clone reacting with the A4.85 MAb is isolated by re-plating and screening the reactive plaque twice. The meningococcal insert DNA from the pure lambda clone (lambda 4.85) is amplified by the polymerase chain reaction (PCR) technique using a kit from Perkin-Elmer/Cetus. The PCR-amplified DNA is cloned into the sequencing vector M13mp19 (Maniatis et al, 1982) and the DNA sequence determined by the dideoxy chain termination method of Sanger et al (Proc. Natl. Acad. Sci. USA 74, 5463-5467 (19877)) using the Sequenase kit (Stratagene, La Jolla, CA).

The cloned meningococcal DNA is labelled with ³²Pby the random primed method with a kit from Boehringer-Mannheim (Indianapolis, IN) and is used in Southern hybridizations (Maniatis et al, 1982) to identify DNA restriction fragments in the FAM20 chromosome adjacent to the DNA cloned in lambda 4.85. Chromosomal Sau3A I fragments of approximately 560 and 1600 bp hybridize to the cloned meningococcal DNA. FAM20 DNA is cleaved with Sau3A I and fractionated on a preparative agarose gel. Two size fractions are isolated, one of 400-700 bp and one of 1400-1800 bp.

The 560 bp Sau3A I fragment is cloned by ligating the 400-700 bp fraction of FAM20-Sau3A I fragments with BamHI-cleaved plasmid pBR322 (Maniatis et al, 1982). The desired clones of the 560 bp fragment are identified by hybridization of bacterial colonies containing recombinant plasmids with ³²P-labelledlambda 4.85 insert DNA (Maniatis et al, 1982). Plasmid DNA (pUNCH201) from a pure colony hybridizing with the DNA probe is prepared and its sequence determined using Sequenase as modified for use in double-strand sequencing (Kraft et al, BioTechniques 6, 544 (1988)). Southern hybridization is used to verify that the cloned fragment is representative of the fragment intact in the FAM20 genome.

To clone the 1600 bp fragment, the ends of the 1400-1800 bp fraction of FAM20-Sau3A I fragments are made blunt by reaction with Klenow enzyme and DNA nucleotides. Synthetic EcoRI linkers are added to these molecules, followed by ligation with EcoRI-cleaved, alkaline-phosphatase-treated lambda ZAP DNA (Stratagene, LaJolla, CA) in accordance with technical information supplied with the lambda ZAP kit. Ligated DNA is packaged into lambda heads using Packagene lambda packaging extracts (Promega). The library of 1400-1800 bp FAM20-Sau3A I fragments is screened with a ³²P-labelledoligonucleotide (SAT1), which is synthesized to correspond to DNA sequences at one end of the lambda 4.85 insert (5' GCCATTGCCACTGTAGATA 3'). A lambda ZAP plaque hybridizing with the SAT1 oligonucleotide is purified as above. The interior portion of this lambda ZAP clone (lambda ZAP202) is "excised" by the addition of helper bacteriophage. The excision results in a multicopy plasmid (pUNCH202) containing the cloned meningococcal insert. Southern hybridization is used to verify that the cloned fragment is representative of the fragment intact in the FAM20 genome. The sequence of the cloned DNA fragment is determined by double-strand sequencing as described above.

The adjoined sequence of pUNCH201, pUNCH202, and the lambda 4.85 insert reveals the presence of an open reading frame that contains the entirety of the cloned DNA. Neither the start or end of the gene is present within this cloned DNA. The DNA sequence is shown as Figure 1. The amino acid sequence predicted by the open reading frame contains 835 amino acids (91kD). The sequence is shown as Figure 2.

As determined by FASTA sequence comparison searches (see above), both the DNA and the deduced polypeptide sequence from this region have a high degree of similarity with a family of hemolysin bacterial toxins. For example, the DNA sequence shown in Figure I exhibits 54% identity with the cya gene (adenylate cyclase) from B. pertussis; 60% identity with the hlyA, hlyB, hlyC and hlyD gene from E. coli (hemolysin); 65% identity with hlyA, hlyB and hlyC gene (hemolysin) from E. coli; 56% identity with the leukotoxin gene from A. actinomycetemcomitans; 56% identity with the hemolysin gene from A. pleuropneumoniae; 60% identity with the leukotoxin gene from P. haemolytica; 62% identity with the A1 leukotoxin gene from P. haemolytica; and 57% identity with protease B gene of E. chrysanthemi.

The amino acid sequence predicted from the DNA sequence exhibited 25%-28% identity with leukotoxin, 22%-28% identity with hemolysin; and 30% identity with adenylate cyclase.

Meningococcal strain FAM20 contains at least two copies of DNA that encode the polypeptides of the invention. This can be demonstrated by digesting genomic DNA with the infrequent cutters BglII, SpeI, NheI, and combinations of NheI and SpeI. Southern blots of the digested DNA separated by pulse field gradient electrophoresis reveal two major bands that hybridize under stringent conditions to gene probes containing fragments of the sequence of the gene that encodes the polypeptide of the invention.

The remainder of the gene encoding the iron-regulated polypeptide of the invention is isolated in a manner similar to that described above for isolating pUNCH201 and pUNCH202. DNA restriction fragments either flanking the ends of the region already cloned or containing the entire region are identified by Southern hybridization using oligonucleotide probes derived from previously determined DNA sequence. These fragments are cloned into either plasmid or bacteriophage vectors as described above for pUNCH201 and pUNCH202. The DNA sequence of newly cloned fragments is determined as above, and reveals when either end of the gene is reached. If the gene is isolated on a single DNA fragment, it is expressed in an in vitro assay to verify that the protein that is encoded by this gene reacts with the A4.85 MAb. If the gene is not cloned intact on a single DNA fragment, it is reconstructed through standard molecular biology techniques to yield the intact gene (Carbonetti, Proc. Natl. Acad. Sci. USA 84, 9084 (1987)).

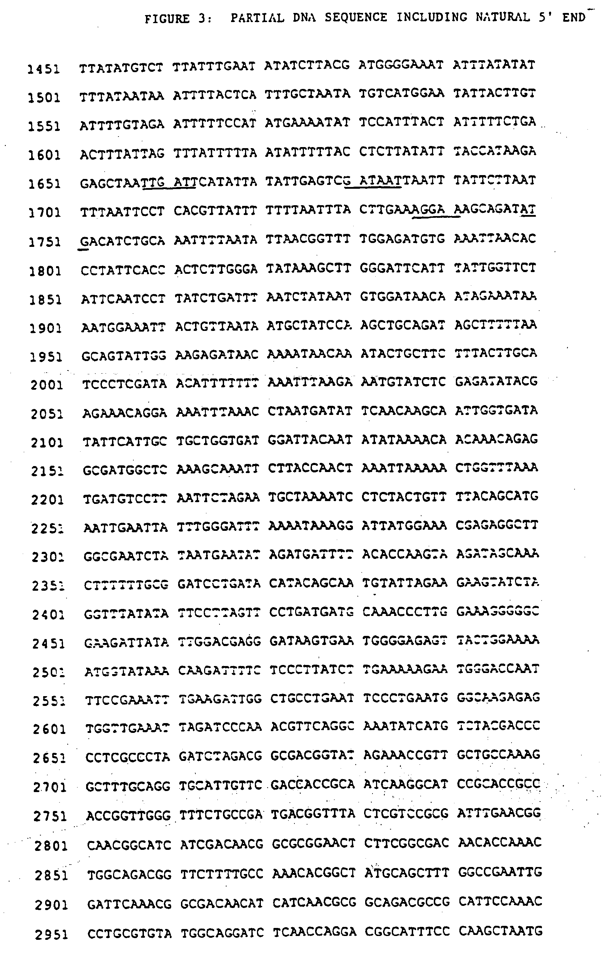

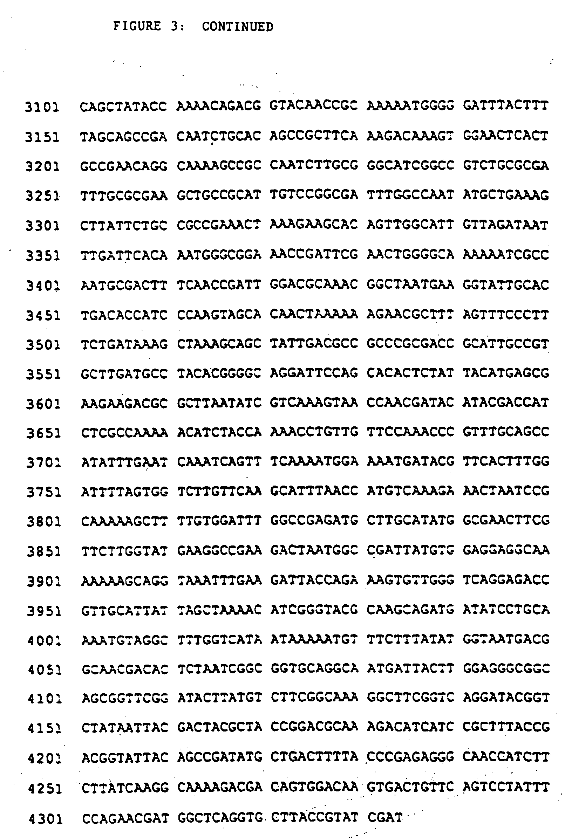

For example, DNA fragments from one of the two copies of the structural genes coding for the polypeptide of the invention were purified from agarose gels, cloned and sequenced. Figure 3 shows the DNA sequence, which is complete in the 5' end of the gene. Underlined in Figure 3 are a typical promoter with a -35 and -10 region, a ribosome binding site, an ATG start site, and a consensus fur box, which is typically found in many gram negative iron-regulated promoters.

### Example 2B. Western Blot and Molecular Weight

The full length polypeptide obtained from meningococcal strain FAM20 exhibits a molecular weight of 230-250 kD when subjected to Western blot analysis. Western blots may be carried out as follows:

Iron-starved whole cells of FAM20 are prepared in accordance with the method of West and Sparling, J. Bacteriol. 169, 3414-3421 (1987). The cells are washed in ice-cold Davis Minimal Medium A (Lederberg, Methods in Med. Res., 3:5 (1950)), immediately cooled on ice, and ruptured in a French pressure cell at 0°C and 20,000 psi. The resulting mixture is centrifuged for 10 minutes at 20,000G, and the pellet solubilized in boiling SDS. The solubilized membrane proteins are separated by standard 7.5% SDS-PAGE in Laemli buffer, which was described by Laemli in Nature 227, 680-685 (1970). The proteins are transferred (16 hours, 80 uA) onto Optibind nitrocellulose membranes (available from from Schleicher & Schuell). The membranes are blocked for 1 hour in 5% BSA in TBS (20mM Tris, 500 mM NaCl, pH 7.5); rinsed for 5 minutes in TBS; incubated for 1 hour with 1:2 dilution of monoclonal antibody A4.85 (see above) in 5% BSA; washed twice for 5 minutes in TBS and 0.05% Tween 20; incubated for 1 hour in a secondary antibody (goat anti-mouse lgG alkaline phosphatase conjugate) diluted in 5% BSA, available from BioRad (dilution = 1:3000) or Sigma (dilution = 1:1000); washed twice for 5 minutes in TBS/Tween; washed again for 5 minutes in TBS; and developed with an alkaline phosphatase substrate comprising 45ul Nitro Blue Tetrazolin, available from Sigma (75 mg/nl); 35 ul 5-bromo-4-chloro-3-indolylphosphate, p-tolnidine salt (50 mg/ml) in 10 ml of carbonate buffer, pH 9.8 (0.1 M NaHCO₃; 1mM MgCl₂).

### Example 3. Assay for Antibody in Sample

A standard ELISA protocol is used to screen for the presence of antibodies against the polypeptide in proteins. Briefly, 96 well microtiter plates are coated with the antigen at concentrations varying from 50-1000ng per well in a high ph (9.6) carbonate buffer. The plates are incubated overnight at 9°C and blocked with 10% normal goat serum for one hour at 37°C. Patient sera is added and titered to determine the endpoint. Control positive and negative sera is added at the same time to quantitate the amount of relevant antibody present in the unknown samples. After a 2-3 hour incubation at 37°C, samples are probed with goat anti-human Ig conjugated to horseradish peroxidase. Positive samples are determined by using TMB.

The invention as claimed is enabled in accordance with the specification and readily available references and starting materials. Nevertheless, the following cell lines have been deposited in the American Type Culture Collection, Bethesda, Maryland on July 12, 1990 in order to facilitate the making and using of the invention:
Meningococcal cell line FAM18 (Accession Number 55071)
Meningococcal cell line FAM20 (Accession Number 55072)
Hybridoma cell line A4.85 (Accession Number HB 10504)

In addition, the following brochures containing useful protocols and information are available in the file history of this specification.
"Predigested Lambda Zap/Eco RI Cloning Kit Instruction Manual," Stratagene, La Jolla, California (November 20, 1987);
"Gigapack Plus" (for packaging recombinant lambda phage), Stratagene, La Jolla, California (April 25, 1988); and
"picoBlue Immunoscreening Kit" Instruction Manual," Stratagene, La Jolla, California (May 19, 1989).

## Claims

1. A segment of at least fifty amino acid residues present in *N. meningitidis,* wherein the amino acid sequence of the segment is different from, but substantially homologous with the amino acid sequence of a segment of a member of the hemolysin family of toxins, wherein the amino acid sequence consists of at least three repeats of the hemolysin consensus sequence LXGGXGNDX, wherein X, independently, represents any single amino acid residue.

2. The segment according to claim 1, wherein the segment is immunogenic.

3. The segment according to any of claims 1-2, wherein the segment is obtained from the sequence shown in Figure 2.

4. The segment according to any of claims 1-3, wherein antibodies against the segment cross-react with at least one other member of the hemolysin family of toxins from other genera of bacteria.

5. The segment according to claim 4, wherein the other genera of bacteria include *Escherichia*, *Serratia*, *Pasteurella*, *Proteus*, *Actinobacillus*, and *Bordetella*.

6. The segment according to claim 4, wherein the other members of the hemolysin family of toxins comprise alpha-hemolysin from *Escherichia coli*; leukotoxin from *Actinobacillus actinomycetemcomitans*; leukotoxin from *Pasteurella haemolytica*; adenylate cyclase from *Bordetella pertussis* and adenylate cyclase from *Bacillus anthraces.*

7. The segment according to claim 4, wherein the other member of the hemolysin family of toxins is alpha-hemolysin from *E. coli*.

8. A method of producing an antigen useful in protecting a mammal from infection by *N. meningitidis* comprising the steps of:
(a) preparing a segment according to any of claims 1-7.
(b) rendering the segment non-toxic to mammals.

9. The method according to claim 8, wherein the mammal is a human.

10. A method of producing a vaccine composition useful in protecting a mammal from infection by *N. meningitidis* comprising the steps of:
(a) preparing a segment according to any of claims 1-7;
(b) rendering the segment non-toxic to mammals; and
(c) combining the non-toxic segment with a pharmaceutically acceptable carrier.

11. The method according to claim 10, wherein the segment is isolated from *N. meningitidis.*

12. The method according to claim 10, wherein a the mammal is a human.

13. A vaccine composition capable of immunizing mammals against infections by *N. meningitidis*, the vaccine composition comprising:
(a) a segment according to any of claims 1-7, and
(b) a pharmaceutically acceptable carrier.

14. The vaccine composition according to claim 13, wherein the segment is present in *N. meningitidis.*

15. The vaccine composition according to claim 14, wherein the segment is present in outer membranes of *N. meningitidis.*

16. The vaccine composition according to any of claims 13-15, wherein the mammal is a human.

17. Monoclonal antibodies raised against a segment according to any of claims 1-7.

18. An isolated nucleic acid molecule that encodes a segment according to any of claims 1-7.

19. A method of detecting the presence of antibodies specific for a member of the hemolysin family of toxins in a sample comprising the steps of:
(a) preparing a segment according to any of claims 1-7; and
- (b) determining whether the segment recognizes an antibody in the sample.

20. A method of detecting the presence of *N. meningitidis* in a sample comprising the steps of:
(a) preparing a probe that recognizes a segment according to any of claims 1-7; and
(b) determining whether the probe recognizes *N. meningitidis* in the sample.

21. The method according to claim 20, wherein the probe is an antibody.

22. The method according to claim 21, wherein the antibody is monoclonal.

23. The method according to claim 20, wherein the probe is a nucleic acid molecule.


SEQUENCE LISTING

<110> University of North Carolina, at Chapel Hill

<120> Antigenic Iron Repressible Proteins from N. Meningitidis Related to the
Hemolysin Family of Toxins

<130> EP08892I-01938/GRI

<140>
<141>

<150> US 19900552649
<151> 1990-07-16

<160> 3

<170> Patentin 3.0

<210> 1
<211> 2721
<212> DNA
<213> cDNA, N-terminal Fragment

<400> 1
GAATTCCCTG AATGGGCAAG AGAGTGGTTG AAATTAGATC CCAAACGTTC AGGCAAATAT 60
CATGTCTACG ACCCCCTCGC CCTAGATCTA GACGGCGACG GTATAGAAAC CGTTGCTGCC 120
AAAGGCTTTG CAGGTGCATT GTTCGACCAC CGCAATCAAG GCATCCGCAC CGCCACCGGT 180
TGGGTTTCTG CCGATGACGG TTTACTCGTC CGCGATTTGA ACGGCAACGG CATCATCGAC 240
AACGGCGCGG AACTCTTCGG CGACAACACC AAACTGGCAG ACGGTTCTTT TGCCAAACAC 300
GGCTATGCAG CTTTGGCCGA ATTGGATTCA AACGGCGACA ACATCATCAA CGCGGCAGAC 360
GCCGCATTCC AAACCCTGCG TGTATGGCAG GATCTCAACC AGGACGGCAT TTCCCAAGCT 420
AATGAATTGC GTACCCTTGA AGAATTGGGT ATCCAATCTT TGGATCTCGC CTATAAAGAT 480
GTAAATAAAA ATCTCGGTAA CGGTAACACT TTGGCTCAGC AAGGCAGCTA TACCAAAACA 540
GACGGTACAA CCGCAAAAAT GGGGGATTTA CTTTTAGCAG CCGACAATCT GCACAGCCGC 600
TTCAAAGACA AAGTGGAACT CACTGCCGAA CAGGCAAAAG CCGCCAATCT TGCGGGCATC 660
GGCCGTCTGC GCGATTTGCG CGAAGCTGCC GCATTGTCCG GCGATTTGGC CAATATGCTG 720
AAAGCTTATT CTGCCGCCGA AACTAAAGAA GCACAGTTGG CATTGTTAGA TAATTTGATT 780
CACAAATGGG CGGAAACCGA TTCGAACTGG GGCAAAAAAT CGCCAATGCG ACTTTCAACC 840
GATTGGACGC AAACGGCTAA TGAAGGTATT GCACTGACAC CATCCCAAGT AGCACAACTA 900
AAAAAGAACG CTTTAGTTTC CCTTTCTGAT AAAGCTAAAG CAGCTATTGA CGCCGCCCGC 960
GACCGCATTG CCGTGCTTGA TGCCTACACG GGGCAGGATT CCAGCACACT CTATTACATG 1020

AGCGAAGAAG ACGCGCTTAA TATCGTCAAA GTAACCAACG ATACATACGA CCATCTCGCC 1080
AAAAACATCT ACCAAAACCT GTTGTTCCAA ACCCGTTTGC AGCCATATTT GAATCAAATC 1140
AGTTTCAAAA TGGAAAATGA TACGTTCACT TTGGATTTTA GTGGTCTTGT TCAAGCATTT 1200
AACCATGTCA AAGAAACTAA TCCGCAAAAA GCTTTTGTGG ATTTGGCCGA GATGCTTGCA 1260
TATGGCGAAC TTCGTTCTTG GTATGAAGGC CGAAGACTAA TGGCCGATTA TGTGGAGGAG 1320
GCAAAAAAAG CAGGTAAATT TGAAGATTAC CAGAAAGTGT TGGGTCAGGA GACCGTTGCA 1380
TTATTAGCTA AAACATCGGG TACGCAAGCA GATGATATCC TGCAAAATGT AGGCTTTGGT 1440
CATAATAAAA ATGTTTCTTT ATATGGTAAT GACGGCAACG ACACTCTAAT CGGCGGTGCA 1500
GGCAATGATT ACTTGGAGGG CGGCAGCGGT TCGGATACTT ATGTCTTCGG CAAAGGCTTC 1560
GGTCAGGATA CGGTCTATAA TTACGACTAC GCTACCGGAC GCAAAGACAT CATCCGCTTT 1620
ACCGACGGTA TTACAGCCGA TATGCTGACT TTTACCCGAG AGGGCAACCA TCTTCTTATC 1680
AAGGCAAAAG ACGACAGTGG ACAAGTGACT GTTCAGTCCT ATTTCCAGAA CGATGGCTCA 1740
GGTGCTTACC GTATCGATGA GATTCATTTC GATAACGGCA AAGTACTGGA TGTTGCCACT 1800
GTCAAAGAAC TGGTACAGCA ATCCACCGAC GGTTCGGACA GATTGTATGC CTACCAATCC 1860
GGAAGTACCT TAAATGGCGG ATTGGGCGAT GACTATCTGT ACGGTGCCGA CGGGAATGAC 1920
CTGCTGAATG GTGATGCAGG CAACGACAGT ATCTACAGTG GCAATGGCAA TGATACGCTC 1980
GATGGAGGAG AAGGCAACGA CGCCCTGTAC GGCTATAATG GTAACGATGC ACTGAATGGT 2040
GGCGAAGGCA ATGATCATTT GAACGGCGAA GACGGTAACG ACACTCTGAT CGGCGGTGCC 2100
GGTAATGATT ACTTGGAGGG CGGCAGCGGT TCGGATACTT ATGTCTTCGG CAAAGGCTTC 2160
GGTCAGGATA CGGTCTATAA TTACGACTAC GCTACCGGAC GCAAAGACAT CATCCGCTTT 2220
ACCGACGGTA TTACAGCCGA TATGCTGACT TTTACCCGAG AGGGCAACCA TCTTCTTATC 2280
AAGGCAAAAG ACGGCAGTGG ACAAGTGACT GTTCAGTCCT ATTTCCAGAA CGATGGCTCA 2340
GGTGCTTACC GTATCGATGA GATTCATTTC GATAACGGCA AAGTACTGGA TGTTGCCACT 2400
GTCAAAAAAC TGGTACAGCA ATCCACCGAC GGTTCGGACA GATTGTATGC CTACCAATCC 2460
GGAAGTACCT AAATGGGCGG ATTGGGCGAT GACTATCTGT ACGGTGCCGA CGGGGATGAC 2520
CTGCTGAATG GTGATGCAGG CAACGACAGT ATCTACAGTG GCAATGGCAA TGATACGCTC 2580
AATGGAGGAG AAGGCAACGA CGCCCTGTAC GGCTATAATG GTAACGATGT ACTGAATGGT 2640
GCCGAAGGCA ATGATCATTT GAACGGCGAA GACGGTAACG ACACTCTAAT CGGCGGTGCA 2700
GGGCAATGAT TACTTGGAGG G 2721

<210> 2
<211> 700
<212> PRT
<213> N-terminal Fragment

<400> 2
Phe Pro Glu Trp Ala Arg Glu Trp Leu Lys Leu Asp Pro Lys Arg Ser
1               5                   10                  15

Gly Lys Tyr His Val Tyr Asp Pro Leu Ala Leu Asp Leu Asp Gly Asp
            20                  25                  30

Gly Ile Glu Thr Val Ala Ala Lys Gly Phe Ala Gly Ala Leu Phe Asp
        35                  40                  45

His Arg Asn Gln Gly Ile Arg Thr Ala Thr Gly Trp Val Ser Ala Asp
    50                  55                  60

Asp Gly Leu Leu Val Arg Asp Leu Asn Gly Asn Gly Ile Ile Asp Asn
65                  70                  75                  80

Gly Ala Glu Leu Phe Gly Asp Asn Thr Lys Leu Ala Asp Gly Ser Phe
                85                  90                  95

Ala Lys His Gly Tyr Ala Ala Leu Ala Glu Leu Asp Ser Asn Gly Asp
            100                 105                 110

Asn Ile Ile Asn Ala Ala Asp Ala Ala Phe Gln Thr Leu Arg Val Trp
        115                 120                 125

Gln Asp Leu Asn Gln Asp Gly Ile Ser Gln Ala Asn Glu Leu Arg Thr
    130                 135                 140

Leu Glu Glu Leu Gly Ile Gln Ser Leu Asp Leu Ala Tyr Lys Asp Val
145                 150                 155                 160

Asn Lys Asn Leu Gly Asn Gly Asn Thr Leu Ala Gln Gln Gly Ser Tyr
                165                 170                 175

Thr Lys Thr Asp Gly Thr Thr Ala Lys Met Gly Asp Leu Leu Leu Ala
            180                 185                 190

Ala Asp Asn Leu His Ser Arg Phe Lys Asp Lys Val Glu Leu Thr Ala
        195                 200                 205

Glu Gln Ala Lys Ala Ala Asn Leu Ala Gly Ile Gly Arg Leu Arg Asp
    210                 215                 220

Leu Arg Glu Ala Ala Ala Leu Ser Gly Asp Leu Ala Asn Met Leu Lys
225                 230                 235                 240

Ala Tyr Ser Ala Ala Glu Thr Lys Glu Ala Gln Leu Ala Leu Leu Asp
                245                 250                 255

Asn Leu Ile His Lys Trp Ala Glu Thr Asp Ser Asn Trp Gly Lys Lys
            260                 265                 270

Ser Pro Met Arg Leu Ser Thr Asp Trp Thr Gln Thr Ala Asn Glu Gly
        275                 280                 285

Ile Ala Leu Thr Pro Ser Gln Val Ala Gln Leu Lys Lys Asn Ala Leu
    290                 295                 300

Val Ser Leu Ser Asp Lys Ala Lys Ala Ala Ile Asp Ala Ala Arg Asp
305                 310                 315                 320

Arg Ile Ala Val Leu Asp Ala Tyr Thr Gly Gln Asp Ser Ser Thr Leu
                325                 330                 335

Tyr Tyr Met Ser Glu Glu Asp Ala Leu Asn Ile Val Lys Val Thr Asn
            340                 345                 350

Asp Thr Tyr Asp His Leu Ala Lys Asn Ile Tyr Gln Asn Leu Leu Phe
        355                 360                 365

Gln Thr Arg Leu Gln Pro Tyr Leu Asn Gln Ile Ser Phe Lys Met Glu
    370                 375                 380

Asn Asp Thr Phe Thr Leu Asp Phe Ser Gly Leu Val Gln Ala Phe Asn
385                 390                 395                 400

His Val Lys Glu Thr Asn Pro Gln Lys Ala Phe Val Asp Leu Ala Glu
                405                 410                 415

Met Leu Ala Tyr Gly Glu Leu Arg Ser Trp Tyr Glu Gly Arg Arg Leu
            420                 425                 430

Met Ala Asp Tyr Val Glu Glu Ala Lys Lys Ala Gly Lys Phe Glu Asp
        435                 440                 445

Tyr Gln Lys Val Leu Gly Gln Glu Thr Val Ala Leu Leu Ala Lys Thr
    450                 455                 460

Ser Gly Thr Gln Ala Asp Asp Ile Leu Gln Asn Val Gly Phe Gly His
465                 470                 475                 480

Asn Lys Asn Val Ser Leu Tyr Gly Asn Asp Gly Asn Asp Thr Leu Ile
                485                 490                 495

Gly Gly Ala Gly Asn Asp Tyr Leu Glu Gly Gly Ser Gly Ser Asp Thr
            500                 505                 510

Tyr Val Phe Gly Lys Gly Phe Gly Gln Asp Thr Val Tyr Asn Tyr Asp
        515                 520                 525

Tyr Ala Thr Gly Arg Lys Asp Ile Ile Arg Phe Thr Asp Gly Ile Thr
    530                 535                 540

Ala Asp Met Leu Thr Phe Thr Arg Glu Gly Asn His Leu Leu Ile Lys
545                 550                 555                 560

Ala Lys Asp Asp Ser Gly Gln Val Thr Val Gln Ser Tyr Phe Gln Asn
                565                 570                 575

Asp Gly Ser Gly Ala Tyr Arg Ile Asp Glu Ile His Phe Asp Asn Gly
            580                 585                 590

Lys Val Leu Asp Val Ala Thr Val Lys Glu Leu Val Gln Gln Ser Thr
        595                 600                 605

Asp Gly Ser Asp Arg Leu Tyr Ala Tyr Gln Ser Gly Ser Thr Leu Asn
    610                 615                 620

Gly Gly Leu Gly Asp Asp Tyr Leu Tyr Gly Ala Asp Gly Asn Asp Leu
625                 630                 635                 640

Leu Asn Gly Asp Ala Gly Asn Asp Ser Ile Tyr Ser Gly Asn Gly Asn
                645                 650                 655

Asp Thr Leu Asp Gly Gly Glu Gly Asn Asp Ala Leu Tyr Gly Tyr Asn
            660                 665                 670

Gly Asn Asp Ala Leu Asn Gly Gly Glu Gly Asn Asp His Leu Asn Gly
        675                 680                 685

Glu Asp Gly Asn Asp Thr Leu Ile Gly Gly Ala Gly
    690                 695                 700

<210> 3
<211> 2884
<212> DNA
<213> cDNA, N-terminal Fragment

<400> 3
TTATATGTCT TTATTTGAAT ATATCTTACG ATGGGGAAAT ATTTATATAT TTTATAATAA 60
ATTTTACTCA TTTGCTAATA TGTCATGGAA TATTACTTGT ATTTTGTAGA ATTTTTCCAT 120
ATGAAAATAT TCCATTTACT ATTTTTCTGA ACTTTATTAG TTTATTTTTA ATATTTTTAC 180
CTCTTATATT TACCATAAGA GAGCTAATTG ATTCATATTA TATTGAGTCG ATAATTAATT 240
TATTCTTAAT TTTAATTCCT CACGTTATTT TTTTAATTTA CTTGAAAGGA AAGCAGATAT 300
GACATCTGCA AATTTTAATA TTAACGGTTT TGGAGATGTG AAATTAACAC CCTATTCACC 360
ACTCTTGGGA TATAAAGCTT GGGATTCATT TATTGGTTCT ATTCAATCCT TATCTGATTT 420
AATCTATAAT GTGGATAACA ATAGAAATAA AATGGAAATT ACTGTTAATA ATGCTATCCA 480
AGCTGCAGAT AGCTTTTTAA GCAGTATTGG AAGAGATAAC AAAATAACAA ATACTGCTTC 540
TTTACTTGCA TCCCTCGATA ACATTTTTTT AAATTTAAGA AATGTATCTC GAGATATACG 600
AGAAACAGGA AAATTTAAAC CTAATGATAT TCAACAAGCA ATTGGTGATA TATTCATTGC 660
TGCTGGTGAT GGATTACAAT ATATAAAACA ACAAACAGAG GCGATGGCTC AAAGCAAATT 720
CTTACCAACT AAATTAAAAA CTGGTTTAAA TGATGTCCTT AATTCTAGAA TGCTAAAATC 780
CTCTACTGTT TTACAGCATG AATTGAATTA TTTGGGATTT AAAATAAAGG ATTATGGAAA 840
CGAGAGGCTT GGCGAATCTA TAATGAATAT AGATGATTTT ACACCAAGTA AGATAGCAAA 900
CTTTTTTGCG GATCCTGATA CATACAGCAA TGTATTAGAA GAAGTATCTA GGTTTATATA 960
TTCCTTAGTT CCTGATGATG CAAACCCTTG GAAAGGGGGC GAAGATTATA TTGGACGAGG 1020
GATAAGTGAA TGGGGAGAGT TACTGGAAAA ATGGTATAAA CAAGATTTTC TCCCTTATCT 1080
TGAAAAAGAA TGGGACCAAT TTCCGAAATT TGAAGATTGG CTGCCTGAAT TCCCTGAATG 1140
GGCAAGAGAG TGGTTGAAAT TAGATCCCAA ACGTTCAGGC AAATATCATG TCTACGACCC 1200
CCTCGCCCTA GATCTAGACG GCGACGGTAT AGAAACCGTT GCTGCCAAAG GCTTTGCAGG 1260
TGCATTGTTC GACCACCGCA ATCAAGGCAT CCGCACCGCC ACCGGTTGGG TTTCTGCCGA 1320
TGACGGTTTA CTCGTCCGCG ATTTGAACGG CAACGGCATC ATCGACAACG GCGCGGAACT 1380
CTTCGGCGAC AACACCAAAC TGGCAGACGG TTCTTTTGCC AAACACGGCT ATGCAGCTTT 1440
GGCCGAATTG GATTCAAACG GCGACAACAT CATCAACGCG GCAGACGCCG CATTCCAAAC 1500
CCTGCGTGTA TGGCAGGATC TCAACCAGGA CGGCATTTCC CAAGCTAATG AATTGCGTAC 1560
CCTTGAAGAA TTGGGTATCC AATCTTTGGA TCTCGCCTAT AAAGATGTAA ATAAAAATCT 1620
CGGTAACGGT AACACTTTGG CTCAGCAAGG CAGCTATACC AAAACAGACG GTACAACCGC 1680
AAAAATGGGG GATTTACTTT TAGCAGCCGA CAATCTGCAC AGCCGCTTCA AAGACAAAGT 1740
GGAACTCACT GCCGAACAGG CAAAAGCCGC CAATCTTGCG GGCATCGGCC GTCTGCGCGA 1800
TTTGCGCGAA GCTGCCGCAT TGTCCGGCGA TTTGGCCAAT ATGCTGAAAG CTTATTCTGC 1860
CGCCGAAACT AAAGAAGCAC AGTTGGCATT GTTAGATAAT TTGATTCACA AATGGGCGGA 1920
AACCGATTCG AACTGGGGCA AAAAATCGCC AATGCGACTT TCAACCGATT GGACGCAAAC 1980
GGCTAATGAA GGTATTGCAC TGACACCATC CCAAGTAGCA CAACTAAAAA AGAACGCTTT 2040

AGTTTCCCTT TCTGATAAAG CTAAAGCAGC TATTGACGCC GCCCGCGACC GCATTGCCGT 2100
GCTTGATGCC TACACGGGGC AGGATTCCAG CACACTCTAT TACATGAGCG AAGAAGACGC 2160
GCTTAATATC GTCAAAGTAA CCAACGATAC ATACGACCAT CTCGCCAAAA ACATCTACCA 2220
AAACCTGTTG TTCCAAACCC GTTTGCAGCC ATATTTGAAT CAAATCAGTT TCAAAATGGA 2280
AAATGATACG TTCACTTTGG ATTTTAGTGG TCTTGTTCAA GCATTTAACC ATGTCAAAGA 2340
AACTAATCCG CAAAAAGCTT TTGTGGATTT GGCCGAGATG CTTGCATATG GCGAACTTCG 2400
TTCTTGGTAT GAAGGCCGAA GACTAATGGC CGATTATGTG GAGGAGGCAA AAAAAGCAGG 2460
TAAATTTGAA GATTACCAGA AAGTGTTGGG TCAGGAGACC GTTGCATTAT TAGCTAAAAC 2520
ATCGGGTACG CAAGCAGATG ATATCCTGCA AAATGTAGGC TTTGGTCATA ATAAAAATGT 2580
TTCTTTATAT GGTAATGACG GCAACGACAC TCTAATCGGC GGTGCAGGCA ATGATTACTT 2640
GGAGGGCGGC AGCGGTTCGG ATACTTATGT CTTCGGCAAA GGCTTCGGTC AGGATACGGT 2700
CTATAATTAC GACTACGCTA CCGGACGCAA AGACATCATC CGCTTTACCG ACGGTATTAC 2760
AGCCGATATG CTGACTTTTA CCCGAGAGGG CAACCATCTT CTTATCAAGG CAAAAGACGA 2820
CAGTGGACAA GTGACTGTTC AGTCCTATTT CCAGAACGAT GGCTCAGGTG CTTACCGTAT 2880
CGAT 2884